(19) Europäisches Patentamt
European Patent Office
Office européen des brevets

(11) Veröffentlichungsnummer: **0 070 453**
**A1**

(12) # EUROPÄISCHE PATENTANMELDUNG

(21) Anmeldenummer: **82106048.0**

(22) Anmeldetag: **07.07.82**

(51) Int. Cl.³: **C 07 C 103/46**, C 07 C 103/737, C 07 C 125/063, C 07 D 295/18, A 01 N 53/00

(30) Priorität: **16.07.81 DE 3128148**

(43) Veröffentlichungstag der Anmeldung: **26.01.83 Patentblatt 83/4**

(84) Benannte Vertragsstaaten: **AT BE CH DE FR GB IT LI NL**

(71) Anmelder: **BASF Aktiengesellschaft, Carl-Bosch-Strasse 38, D-6700 Ludwigshafen (DE)**

(72) Erfinder: **Buschmann, Ernst, Dr., Georg-Ludwig-Krebs-Strasse 10, D-6700 Ludwigshafen (DE)**
Erfinder: **Schulz, Guenter, Dr., An der Froschlache 3, D-6700 Ludwigshafen (DE)**
Erfinder: **Zeeh, Bernd, Dr., Thorwaldsenstrasse 5, D-6700 Ludwigshafen (DE)**
Erfinder: **Jung, Johann, Dr.Dipl.Landwirt, Hardenburgstrasse 19, D-6703 Limburgerhof (DE)**

(54) **N-Acylcyclopropylamine, Verfahren zu ihrer Herstellung und diese Verbindungen enthaltende Mittel zur Regulierung des Pflanzenwachstums.**

(57) N-Acylcyclopropylamine der Formel

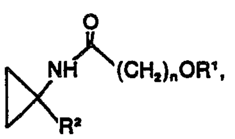

in der $R^1$ einen Alkyl-, Alkoxyalkyl-, Allyl-, Aralkyl- oder Arylrest, n 0 oder 1 und $R^2$ einen Carboxyrest bedeuten, der in Form der entsprechenden Salze, Ester oder Amide vorliegen kann und Mittel zur Regulierung des Pflanzenwachstums, die diese Verbindungen enthalten.

EP 0 070 453 A1

N-Acylcyclopropylamine, Verfahren zu ihrer Herstellung und diese Verbindungen enthaltende Mittel zur Regulierung des Pflanzenwachstums

Die vorliegende Erfindung betrifft neue N-Acylcyclopropylamine und Mittel zur Regulierung des Pflanzenwachstums, die diese Verbindungen enthalten, sowie Verfahren zur Herstellung der Verbindungen.

Es ist bekannt, daß Aminocyclopropancarbonsäure (ACC) pflanzenwachstumsregulierende Eigenschaften aufweist. [T. Boller, R. C. Herner, H. Kende, Planta 145, 293-303 (1979)].

Es ist ferner bekannt, N,N,N-Trimethyl-N-2-chlorethyl--ammoniumchlorid (CCC) als Wachstumsregulator für Pflanzen zu verwenden (US-PS 3,156,554).

Es wurde nun gefunden, daß N-Acylcyclopropylamine der Formel

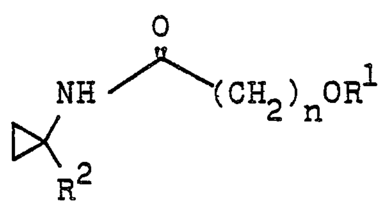

,

in der $R^1$ einen Alkyl-, Alkoxyalkyl, Alkenyl, Aralkyl- oder Arylrest, n 0 oder 1 und $R^2$ einen Carboxyrest bedeuten, der in Form der entsprechenden Na-, K-, Ca- oder Ammoniumsalze oder Aryl- oder Alkylester oder unsubstituierten, mono- oder disubstituierten Amide vorliegen kann, starke pflanzenwachstumsregulierende Eigenschaften haben.

Mu/P

$R^1$ bedeutet beispielsweise $C_1$-$C_4$-Alkyl wie Methyl, Ethyl, Propyl, Isopropyl, n-Butyl, Isobutyl, tert.-Butyl, $C_3$-Alkoxyalkyl wie 2-Methoxyethyl, $C_3$-$C_4$-Alkenyl wie Allyl, Crotyl, Phenyl, Halogenphenyl wie 4-Chlorphenyl, $C_1$-$C_4$-Alkylphenyl wie 4-Methylphenyl, Dihalogenphenyl wie 2,4-Dichlorphenyl, 4-Nitrophenyl, Benzyl, Halogenbenzyl wie 4-Chlorbenzyl, Dihalogenbenzyl, wie 2,4-Dichlorbenzyl.

$R^2$ bedeutet beispielsweise $CO_2H$, die entsprechenden Ester, wie die $C_3$-$C_4$-Alkylester, die entsprechenden Salze, wie die Alkalisalze, d.h. die Salze mit Na, K, Ca oder die Ammoniumsalze, wie die $NH_4$-, Dialkyl-$C_1$-$C_4$-ammonium-, Piperidinium-, Dimethylpiperidinium-, Pyrrolidinium-, Morpholiniumsalze und unsubstituierte oder gegebenenfalls niederalkylsubstituierte Säureamide, wie die $CONH_2$- oder $CONHCH_3$-Verbindungen.

Überraschenderweise zeigen die neuen Wirkstoffe eine wesentlich stärkere pflanzenwachstumsregulierende Wirkung als die bekannte Aminocyclopropancarbonsäure (ACC).

Ausgangsverbindung für die neuen N-Acylcyclopropylamine ist Aminocyclopropancarbonsäure (ACC), deren Herstellung beschrieben ist von D. H. Rich und J. P. Tam, Synthesis 1978, 46 und I. Bregovec und T. Jakovčić, Monatshefte 103, 288-291 (1972). Als Ausgangsverbindungen verwendbar sind auch die Ester der Formel I

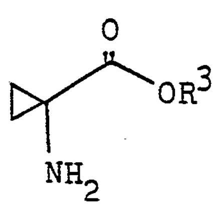 I,

in der $R^3$ Alkyl oder Aryl bedeutet. Diese Ester sind teilweise bekannt durch C. K. Ingold, S. Sako und J. F.

Thorpe, J. Chem. Soc. 1922 (1177-1198) und U. Schöllkopf, D. Hoppe und R. Jentsch, Chem. Ber. 108, 1580-1592 (1975). Noch nicht beschriebene Ester der Formel I können ohne Schwierigkeiten entsprechend den bekannten Beispielen hergestellt werden.

Das folgende Schema beschreibt, wie die neuen Substanzen mit allgemein bekannten Reaktionen hergestellt werden können:

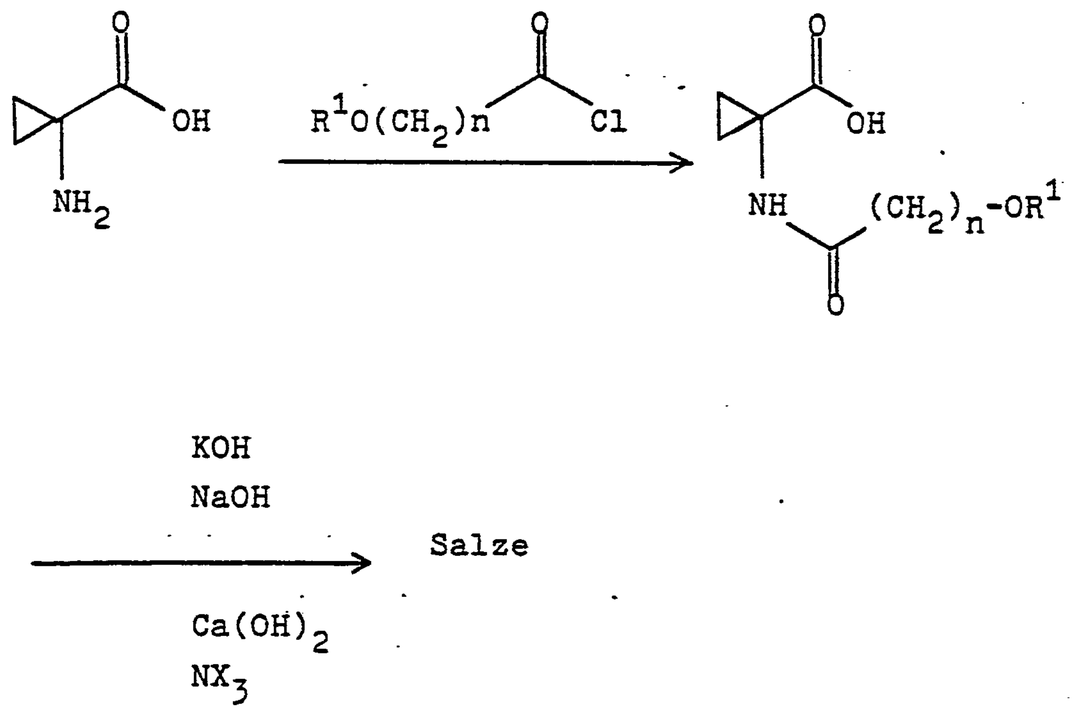
KOH
NaOH
Salze
Ca(OH)2
NX3

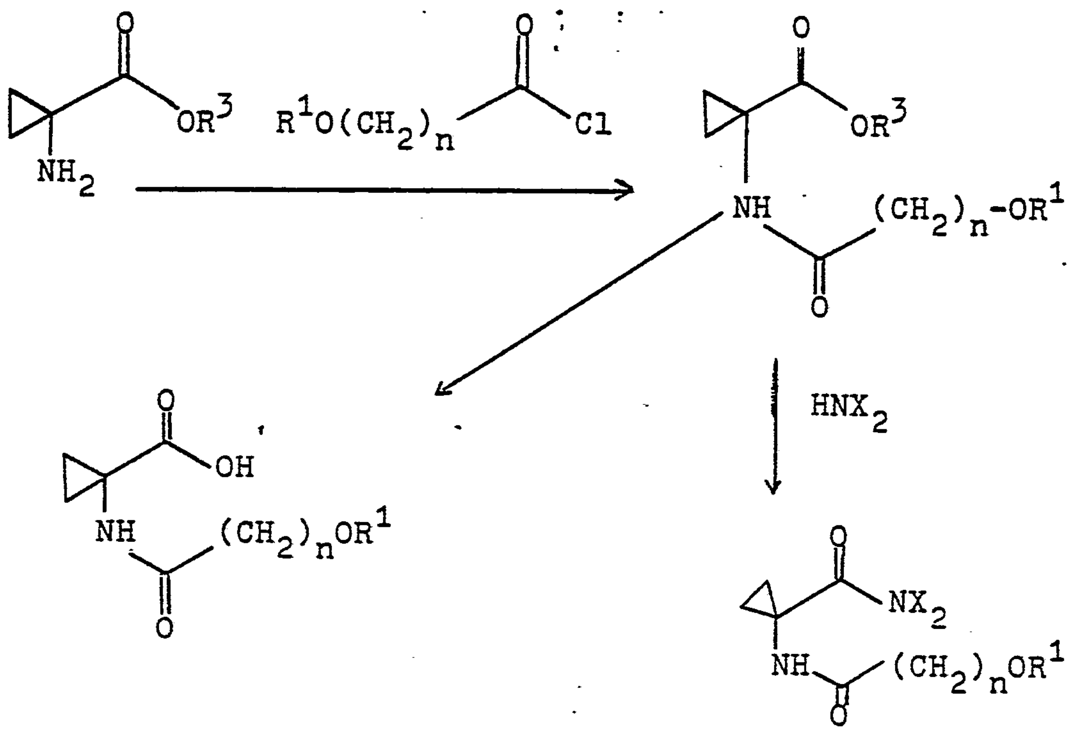
HNX2

$R^1$, $R^3$ und n haben die obengenannten Bedeutungen. $NX_3$ steht für $NH_3$, ein primäres, sekundäres oder tertiäres Amin, z.B. $NH(CH_3)_2$, $N(CH_3)_3$, $HNCH_2C_6H_5$, Piperidin, Methylpiperidin, Dimethylpiperidin, Pyrrolidin, Morpholin, Hexamethylenimin, Ethanolamin, Diethanolamin. $HNX_2$ steht für $NH_3$, ein primäres oder sekundäres Amin, z.B. $NH(CH_3)_2$, $H_2NCH_3$, $HN(C_2H_5)_2$, Pyrrolidin, Piperidin, Morpholin.

Die folgende Vorschrift und Beispiele erläutern die Herstellung der Vorprodukte und der neuen Verbindungen:

Vorschrift 1

Aminocyclopropancarbonsäuremethylester (Verbindung a)
181 g Aminocyclopropancarbonsäuremethylesterhydrochlorid werden in 1000 $cm^3$ Wasser gelöst. Man gibt 100 g $NaHCO_3$ portionsweise zu, extrahiert mehrfach mit Ether, trocknet über $Na_2SO_4$, engt ein und destilliert. Man erhält 100 g (Verbindung a). Sdp. 40 bis 46°C/0,6 mbar.

Beispiel 1

N-Carbomethoxy-aminocyclopropancarbonsäuremethylester (Verbindung Nr. 14)

Zu einer Lösung von 11,5 g (Verbindung a), 8 g Pyridin, 0,4 g 4-Dimethylaminopyridin in 200 ml Ether wird unter Eiskühlung 9,4 g Chlorameisensäuremethylester in 50 ml Ether zugetropft. Nach 14 h Rühren bei 20°C wird filtriert, eingeengt und destilliert. Man erhält 9 g (Verbindung Nr.14). Sdp. 90-95°C/0,5 mbar.

Beispiel 2

N-Carbophenoxy-aminocyclopropancarbonsäuremethylester (Verbindung Nr. 18)

Zu einer Lösung von 11,5 g (Verbindung a) 8 g Pyridin, 0,4 g 4-Dimethylaminopyridin in 200 ml Ether wird unter Eiskühlung 15,6 g Chlorameisensäurephenylester zugetropft. Nach 14 h Rühren bei 20°C wird die etherische Phase mehrfach mit Wasser gewaschen, über $Na_2SO_4$ getrocknet und eingeengt. Der Rückstand wird mit n-Pentan verrieben, abgesaugt und getrocknet. Man erhält 14,5 g (Verbindung Nr. 18) Fp. 83°C.

Beispiel 3

N-Carbo-(butoxy)-aminocyclopropancarbonsäure (Verbindung Nr. 7)

6,5 g N-Carbo-(butoxy)-aminocyclopropancarbonsäuremethylester und 1,8 g NaOH werden in 10 ml $H_2O$ und 50 ml Ethanol gelöst. Man rührt 2 h bei 50°C, dampft die Hauptmenge des Ethanols ein, gibt 50 ml Wasser zu und säuert mit konz. HCl an. Dabei fällt (Verbindung Nr. 7) aus. Man saugt ab, wäscht mit wenig Wasser und trocknet im Vakuum. Ausbeute 4 g (Verbindung Nr. 7), Fp. 110°C.

Beispiel 4

N-Methoxyacetyl-aminocyclopropancarbonsäureamid (Verbindung Nr. 28)

6 g N-Methoxyacetyl-aminocyclopropancarbonsäuremethylester (Verbindung Nr. 23) werden in 50 ml wäßriger konzentrierter $NH_3$-Lösung gelöst. Man rührt 14 h bei 20°C, engt ein

und trocknet im Vakuum. Man erhält 5,5 g (Verbindung Nr. 28), Fp. 128°C.

Wie oben beschrieben, werden die folgenden neuen Substanzen hergestellt:

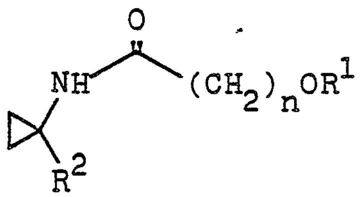

| Nr. | $R^1$ | $R^2$ | n | Fp °C Sdp. °C/mbar |
|---|---|---|---|---|
| 1 | $CH_3$ | $CO_2H$ | 0 | 135° |
| 2 | $C_2H_5$ | $CO_2H$ | 0 | 111° |
| 3 | n-$C_3H_7$ | $CO_2H$ | 0 | |
| 4 | t-Butyl | $CO_2H$ | 0 | |
| 5 | $C_6H_5$ | $CO_2H$ | 0 | |
| 6 | $CH_2CH=CH_2$ | $CO_2H$ | 0 | 117° |
| 7 | n-$C_4H_9$ | $CO_2H$ | 0 | 110° |
| 8 | $(CH_2)_2OCH_3$ | $CO_2H$ | 0 | 105° |
| 9 | $CH_3$ | $CO_2H$ | 1 | |
| 10 | $CH_3$ | $CO_2Na$ | 0 | 190° |
| 11 | $C_2H_5$ | $CO_2K$ | 0 | |
| 12 | $CH_3$ | $CO_2NH_4$ | 0 | |
| 13 | $CH_3$ | $CO_2Na$ | 1 | |
| 14 | $CH_3$ | $CO_2CH_3$ | 0 | 90-95°/0,5 |
| 15 | $C_2H_5$ | $CO_2CH_3$ | 0 | 110°/0,3 |

| Nr. | $R^1$ | $R^2$ | n | Fp °C<br>Sdp. °C/mbar |
|---|---|---|---|---|
| 16 | $n-C_4H_9$ | $CO_2CH_3$ | 0 | 118-123°/0,3 |
| 17 | t-Butyl | $CO_2CH_3$ | 0 | |
| 18 | $C_6H_5$ | $CO_2CH_3$ | 0 | 83° |
| 19 | Allyl | $CO_2CH_3$ | 0 | 116-119°/0,3 |
| 20 | $(CH_2)_2OCH_3$ | $CO_2CH_3$ | 0 | 128-132°/0,3 |
| 21 | $CH_3$ | $CO_2C_2H_5$ | 0 | |
| 22 | $CH_3$ | $CO_2C_6H_5$ | 0 | |
| 23 | $CH_3$ | $CO_2CH_3$ | 1 | 100-110°/0,6 |
| 24 | $CH_3$ | $CONH_2$ | 0 | |
| 25 | $CH_3$ | $CONHCH_3$ | 0 | |
| 26 | $CH_3$ | $CON(CH_3)_2$ | 0 | |
| 27 | $CH_3$ | CON 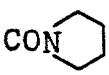 | 0 | |
| 28 | $CH_3$ | $CONH_2$ | 1 | 128° |
| 29 | $CH_3$ | $CON(CH_3)_2$ | 1 | |
| 30 | $CH_2C_6H_5$ | $CO_2C_2H_5$ | 0 | 56° |
| 31 | $CH_2C_6H_5$ | $CO_2H$ | 0 | 158° |
| 32 | $CH_3$ | $CO_2H_2N$ 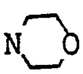 | 0 | 133° |

Die neuen Wirkstoffe greifen in den Stoffwechsel der Pflanzen ein und können deshalb als Wachstumsregulatoren eingesetzt werden.

Für die Wirkungsweise von Pflanzenwachstumsregulatoren gilt nach den bisherigen Erfahrungen, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z.B. Sonnenscheindauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von der angewendeten Konzentrationen der aktiven Substanz.

In jedem Falle sollen Wachstumsregulatoren die Kulturpflanzen in gewünschter Weise positiv beeinflussen.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den neuen Verbindungen läßt sich das vegetative Wachstum der Pflanzen stark hemmen, was sich insbesonders in einer Reduzierung des Längenwachstums äußert. Die behandelten Pflanzen weisen demgemäß einen gedrungenen Wuchs auf; außerdem ist einde dunklere Blattfärbung zu beobachten.

Als vorteilhaft für die Praxis erweist sich z.B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachten Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Ein weiterer Mechanismus der Ertragssteigerung mit Wachstumshemmern beruht darauf, daß die Nährstoffe in stärkerem Maße der Blüten- und Fruchtbildung zugutekommen, während das vegetative Wachstum eingeschränkt wird. Ferner kann so wegen der relativ geringen Blatt- bzw. Pflanzenmasse dem Befall mit verschiedenen, insbesondere pilzlichen Krankheiten vorgebeugt werden.

Die Hemmung des vegetativen Wachstums ermöglicht außerdem bei vielen Kulturpflanzen eine dichtere Bepflanzung, so daß ein Mehrertrag, bezogen auf die Bodenfläche erzielt werden kann. Die erfindungsgemäßen Verbindungen eignen sich besonders zur Hemmung des vegetativen Wachstums bei Kulturpflanzen wie Soja, Sonnenblumen, Erdnüssen, Raps, Zierpflanzen, Baumwolle, Reis und Gräsern.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die neuen Wirkstoffe können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,001 bis 12 kg/ha bevorzugt 0,01 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung oder in einer Lösung von Wasser unter Zusatz von mit Wasser mischbaren organischen Lösungsmitteln wie Methanol oder anderen niederen Alkoholen, Aceton, Dimethylformamid

oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Die erfindungsgemäßen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d.h. die Wirksamkeit des Kombinationsproduktes ist größer als die addierten Wirksamkeiten der Einzelkomponenten.

In den nachfolgenden Versuchen wird die Wirkung der erfindungsgemäß verwendbaren Stoffe als Pflanzenwachstumsregulatoren dargestellt, ohne die Möglichkeit weiterer Anwendungen als Wachstumsregulatoren auszuschließen.

Die folgenden Versuche erläutern die biologische Wirkung der neuen Verbindungen.

Vergleichssubstanzen:

A) 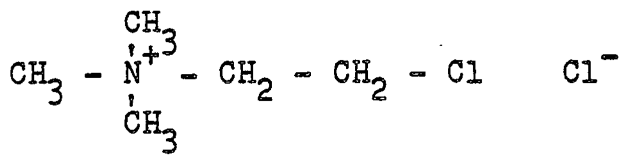 = CCC

B) 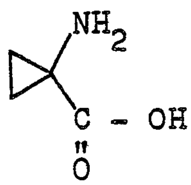 = ACC

Versuch 1

Prüfung auf wachstumsregulierende Wirkung (WR)

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Nachlaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanzen dienten CCC und ACC.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

In diesem Versuch zeigten die Wirkstoffe 1 und 2 eine wesentlich stärkere Reduzierung des Längenwachstums bei Sonnenblumen im Nachauflaufverfahren bei Aufwandmengen von 1,5 und 6 mg Wirkstoff je Gefäß als die Verbindungen A und B.

Versuch 2

Prüfung auf Erhöhung des Ethylenspiegels bei behandelten Pflanzen

Zur Bestimmung der Ethylenentwicklung wurden die Laubblätter von Pflanzen, die wie oben beschrieben angezogen und behandelt worden waren, nach einer Versuchsdauer von 18 bis 22 Tagen in Segmente zerschnitten. Von dem zerkleinerten Grünmaterial wurde ein Teil in Erlenmeyerkolben mit gasdichtem Membranverschluß eingewogen. Nach einer Verweildauer von 24 Stunden wurden 1000 $\mu$l der Gasphase gaschromatographisch auf ihren Ethylengehalt untersucht.

In diesem Versuch bewirkten die Verbindungen 1, 2, 6, 7, 8, 14, 23 und 30, bei Aufwandmengen von 1,5 und 6 mg Wirkstoff je Gefäß, bei Sonnenblumen eine starke Entwicklung von Ethylen.

Dieser Test ist in besonderem Maße geeignet, um die wachstumsregulierenden Eigenschaften der neuen Verbindungen zu verdeutlichen.

Das Pflanzenhormon Ethylen greift in zahlreiche Prozesse bei der Entwicklung der Pflanzen ein. Eine Erhöhung der Ethylenbiosynthese, wie sie mit den neuen Substanzen erzielt wird, erlaubt es, diese Prozesse zu steuern. Als Beispiele seien hier genannt: Fruchtablösung, Reifebeschleunigung von Früchten und Blättern, Blühinduktion,

Samenkeimung, Fruchtausdünnung, Stimulation des Latexflusses, Geschlechtsbeeinflussung und Wuchshemmung.

Beispiel I

20 Teile des Wirkstoffs gemäß Beispiel 1 werden mit 12 Teilen Calciumsalz der Dodecylbenzolsulfonsäure, 8 Teilen Fettalkoholpolyglykolether, 2 Teilen Natriumsalz eines Phenolsulfonsäure-Harnstoff-Kondensats, 68 Teilen eines paraffinischen Mineralöls innig vermischt. Man erhält eine stabile ölige Dispersion.

Beispiel II

3 Gewichtsteile des Wirkstoffs gemäß Beispiel 2 werden mit 97 Gewichtsteilen feinteiligem Kaolin innig vermischt. Man erhält auf diese Weise ein Stäubemittel, das 3 Gewichtsprozent des Wirkstoffs enthält.

Beispiel III

30 Gewichtsprozent des Wirkstoffs gemäß Beispiel 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung mit guter Haftfähigkeit.

Beispiel IV

40 Gewichtsteile des Wirkstoffs gemäß Beispiel 2 werden mit 10 Teilen Natriumsalz eines Phenolsulfonsäure-Formaldehyd-Kondensats, 2 Teilen Kieselsäuregel und 48 Teilen Wasser innig vermischt. Man erhält eine stabile wäßrige

Dispersion. Durch Verdünnen mit Wasser erhält man eine wäßrige Dispersion.

Beispiel V

20 Gewichtsteile des Wirkstoffs gemäß Beispiel 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion.

Beispiel VI

20 Gewichtsteile des Wirkstoffs gemäß Beispiel 1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

Beispiel VII

80 Gewichtsteile des Wirkstoffs gemäß Beispiel 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

## Patentansprüche

1. N-Acylcyclopropylamin der Formel

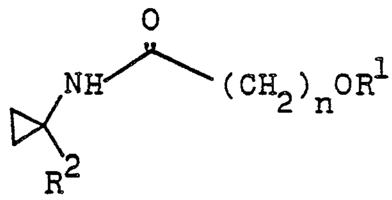

,

in der $R^1$ einen Alkyl-, Alkoxyalkyl-, Alkenyl-, Aralkyl-, oder Arylrest, n 0 oder 1 und $R^2$ einen Carboxyrest bedeuten, der in Form der entsprechenden Na-, K-, Ca- oder Ammoniumsalze, Aryl- oder Alkylester oder unsubstituierten, mono- oder disubstituierten Amide vorliegen kann.

2. N-Acylcyclopropylamine gemäß Anspruch 1 mit der Formel

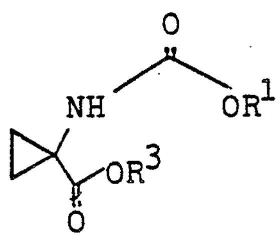

,

in der $R^1$ die im Anspruch 1 genannten Bedeutungen hat und $R^3$ Wasserstoff, Alkyl oder Aryl bedeutet.

3. N-Acylcyclopropylamine, nämlich
1-Carbomethoxy-N-methoxyacetyl-cyclopropylamin,
1-Carboxy-N-carbomethoxy-cyclopropylamin
1-Carbomethoxy-N-carbomethoxy-cyclopropylamin oder
1-Carboxy-N-carboethoxy-cyclopropylamin.

4. Verfahren zur Herstellung eines N-Acylpropylamins gemäß Anspruch 1, <u>dadurch gekennzeichnet</u>, daß man ein Cyclopropanaminosäurederivat der Formel

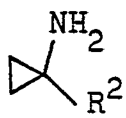 ,

in der $R^2$ die in Anspruch 1 genannten Bedeutungen hat mit einer Verbindung der Formel $Cl-CO-(CH_2)_nOR^1$, in der $R^1$ und n die in Anspruch 1 genannten Bedeutungen haben, umsetzt.

5. Mittel zur Regulierung des Pflanzenwachstums enthaltend ein N-Acylcyclopropylamin gemäß Anspruch 1.

6. Mittel zur Regulierung des Pflanzenwachstums, enthaltend inerte Zusatzstoffe und ein N-Acylcyclopropylamin gemäß Anspruch 1.

7. Verfahren zur Regulierung des Pflanzenwachstums, <u>dadurch gekennzeichnet</u>, daß man eine wirksame Menge eines N-Acylcyclopropylamins gemäß Anspruch 1 auf Pflanzen und/oder deren Lebensraum einwirken läßt.

8. Verfahren zur Herstellung eines Mittels zur Regulierung des Pflanzenwachstums gemäß Anspruch 6, <u>dadurch gekennzeichnet</u>, daß man ein N-Acylcyclopropylamin gemäß Anspruch 1 mit oberflächenaktiven Verbindungen und/oder Streckmitteln und/oder Synergisten mischt.

9. Verwendung von N-Acylcyclopropylamin gemäß Anspruch 1 zur Regulierung des Pflanzenwachstums.

Europäisches Patentamt

**EUROPÄISCHER RECHERCHENBERICHT**

0070453 Nummer der Anmeldung

EP 82 10 6048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 030 287 (BAYER) *Patentansprüche* | 1-9 | C 07 C 103/46<br>C 07 C 103/737<br>C 07 C 125/063<br>C 07 D 295/18<br>A 01 N 53/00 |
| X | SYNTHESIS, Nr. 1, Januar 1978, Seite 46 D.H.RICh et al.: "A convenient synthesis of the Amino Acid, 1-Amino-cyclopropane-1-carboxylic acid". *Seite 46* | 1-2 | |
| X | EP-A-0 025 141 (BAYER) *Seiten 2-3* | 1-2 | |

-----

**RECHERCHIERTE SACHGEBIETE (Int. Cl. 3)**

C 07 C 103/00
C 07 C 125/00
C 07 D 295/00
A 01 N 53/00

Der vorliegende Recherchenbericht wurde für alle Patentansprüche erstellt.

| Recherchenort | Abschlußdatum der Recherche | Prüfer |
|---|---|---|
| DEN HAAG | 15-10-1982 | MOREAU J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument